# FASCICULE DE BREVET EUROPEEN

(11) **EP 2 888 990 B1**
(45) Date de publication et mention de la délivrance du brevet: **28.11.2018**
(21) Numéro de dépôt: 14200532.1
(22) Date de dépôt: 30.12.2014
(51) Int. Cl.: A61B 1/00, A61B 1/07, A61B 1/06, A61B 5/00, A61B 10/02, A61B 10/04, A61B 10/00

(54) **Dispositif de prélèvement d'un tissu biologique**
Vorrichtung zur Entnahme eines biologischen Gewebes
Device for sampling a biological tissue

(30) Priorité: 30.12.2013 FR 1363695
(43) Date de publication de la demande: 01.07.2015
(73) Titulaire: Commissariat à l'Énergie Atomique et aux Énergies Alternatives, 75015 Paris (FR); Centre Hospitalier Universitaire Grenoble Alpes, 38700 La Tronche (FR); Université Grenoble Alpes, 38400 Saint-Martin-d'Hères (FR)
(72) Inventeur: Dreyfus, Matthieu, 38000 Grenoble (FR); Berger, François, 38240 Meylan (FR); Bouamrani, Ali, 38000 Grenoble (FR); Mombrun, Adrien, 38000 Grenoble (FR)
(74) Mandataire: Regimbeau

(56) Documents cités:
- WO-A1-2013/091090
- WO-A1-2013/098703
- US-A- 3 945 375

## Description

### DOMAINE DE L'INVENTION

La présente invention concerne un dispositif de prélèvement d'un tissu biologique.

### ARRIERE PLAN DE L'INVENTION

Le prélèvement de tissus biologiques dans certains organes d'un être vivant se révèle particulièrement complexe du fait des risques de lésion de l'organe dans lequel est effectué le prélèvement.

Tel est le cas par exemple des tumeurs du cerveau, qui sont peu accessibles à la biopsie compte tenu des risques encourus en termes de lésions cognitives irréversibles.

Le document WO 2006/082344 décrit un dispositif de prélèvement minimalement invasif, qui comprend une tige et une surface de capture portée par la tige, destinée à être appliquée contre un tissu biologique.

Le document WO 2013/098703 décrit un dispositif similaire, dans lequel la surface de capture comporte un matériau nanoporeux, et en particulier du silicium nanoporeux.

Par simple contact entre la surface de capture - qui est avantageusement micro-structurée ou poreuse - et le tissu, on obtient une capture de cellules et de macromolécules dans des territoires du système nerveux inaccessibles par chirurgie.

Compte tenu du fait que la taille de la tumeur peut être faible, il est nécessaire que la surface de capture soit amenée de manière précise au niveau de la tumeur afin de prélever les cellules et macromolécules d'intérêt.

Cependant, il est relativement complexe de localiser précisément la zone anatomique où est effectué le prélèvement au moyen de ce dispositif, le praticien ne pouvant visualiser directement l'organe dans lequel il est introduit.

A cet effet, une possibilité est de rendre le dispositif de prélèvement compatible avec l'imagerie par résonance magnétique (IRM) - c'est-à-dire typiquement en le réalisant dans des matériaux non magnétiques -, ce qui permet d'observer par cette technique d'imagerie l'introduction du dispositif de prélèvement dans l'organe et de vérifier l'emplacement de la surface de capture.

Cependant, la mise en oeuvre du prélèvement sous IRM pose des difficultés en termes de disponibilité de l'équipement.

Il serait donc souhaitable de pouvoir procéder à un prélèvement de tissus biologiques dans une zone précise d'un organe en s'affranchissant de l'imagerie par résonance magnétique.

Le document WO 2013/091090 divulgue un dispositif de biopsie comprenant une canule qui comporte une fenêtre pourvue de bords tranchants pour aspirer puis découper l'échantillon à prélever. Un faisceau de fibres est agencé dans le conduit intérieur de ladite canule.

### BREVE DESCRIPTION DE L'INVENTION

Un but de l'invention est de concevoir un dispositif de prélèvement de tissus biologiques minimalement invasif qui permette de localiser avec une précision améliorée l'emplacement de la zone dans laquelle est effectué le prélèvement, sans impliquer de système d'imagerie externe tel que l'IRM.

Conformément à l'invention, il est proposé un dispositif de prélèvement d'un tissu biologique comprenant :
- une tige s'étendant selon un axe longitudinal entre une extrémité proximale et une extrémité distale,
- une surface de capture portée par la tige, destinée à être appliquée contre un tissu biologique,
- une fenêtre d'observation transparente à la lumière visible, agencée dans la surface externe de la tige,
- un logement s'étendant dans la tige, parallèlement à l'axe longitudinal, de l'extrémité proximale jusqu'à la fenêtre d'observation, ledit logement étant apte à recevoir un faisceau de fibres optiques,
ledit dispositif étant caractérisé en ce que la surface de capture est nanoporeuse ou présente des protubérances adaptées pour prélever un tissu biologique par microabrasion.

De manière avantageuse, la fenêtre d'observation et la surface de capture sont agencées à une même distance de l'extrémité distale de la tige et angulairement espacées.

Ledit logement du faisceau de fibres optiques comprend une rainure agencée dans la surface externe de la tige.

Selon un mode de réalisation, le dispositif comprend en outre :
- un faisceau de fibres optiques disposé dans le logement de la tige de telle sorte qu'une extrémité distale dudit faisceau soit agencée au voisinage de la fenêtre d'observation et
- un système optique de renvoi adapté pour réaliser un couplage optique entre l'extrémité distale du faisceau et la fenêtre d'observation.

Selon une forme d'exécution, le système optique de renvoi comprend une lame réfléchissante orientée d'un angle compris entre 40 et 50° par rapport à l'axe longitudinal de la tige.

De manière avantageuse, ladite lame réfléchissante fait partie d'un prisme.

Selon un mode de réalisation, le prisme comprenant ladite lame réfléchissante est solidaire de l'extrémité distale du faisceau de fibres optiques.

De manière alternative, le système optique de renvoi consiste en une extrémité distale biseautée des fibres optiques formant le faisceau.

Le dispositif peut en outre comprendre un tube guide dans lequel la tige est apte à coulisser, ledit tube guide comprenant une ouverture latérale agencée de telle sorte que lorsque la tige est introduite dans le tube guide la surface de capture et la fenêtre d'observation viennent en vis-à-vis de ladite ouverture latérale selon l'orientation angulaire respective du tube guide et de la tige.

La surface de capture est avantageusement située dans une région distale de la tige.

Selon un mode de réalisation, la surface de capture est revêtue d'une couche de fonctionnalisation.

La tige est par exemple en inox ou en PEEK.

Selon une forme d'exécution avantageuse, la tige présente une section décroissante de son extrémité proximale vers son extrémité distale.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue en coupe d'un dispositif de prélèvement selon un mode de réalisation,
- la figure 2 est une vue de face de l'extrémité proximale de la tige du dispositif de prélèvement,
- la figure 3 est une vue en perspective de côté de la tige,
- la figure 4 est une vue en perspective de la région de la fenêtre d'observation dans la tige,
- la figure 5 est une vue en perspective de la tige dans le tube guide, en position d'observation des tissus,
- la figure 6 est une vue en perspective de la tige dans le tube guide, en position de prélèvement d'un tissu biologique,
- la figure 7 est une vue en perspective de la tige selon une forme d'exécution particulière de l'invention.

### DESCRIPTION DETAILLEE DE L'INVENTION

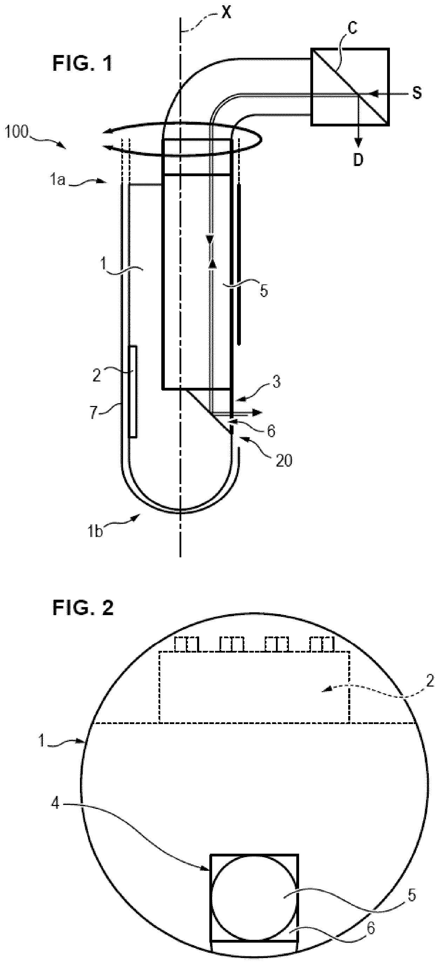

La figure 1 est une vue en coupe d'un mode de réalisation d'un dispositif 100 de prélèvement de tissus biologiques conforme à l'invention.

Le dispositif 100 comprend une tige 1 qui s'étend selon un axe longitudinal X entre une extrémité proximale 1a et une extrémité distale 1b. Par convention, on désigne par « proximal » le côté le plus proche de la main du praticien et par « distal » le côté le plus éloigné, destiné à entrer en contact avec les tissus.

La tige 1 est réalisée en un matériau biocompatible. La tige 1 peut ainsi être réalisée en acier inoxydable, ou encore en un matériau plastique, tel que le PEEK qui présente, par rapport à l'inox, d'être compatible avec l'IRM.

Le diamètre de la tige est typiquement compris entre 500 µm et 2000 µm, de préférence entre 800 µm et 1200 µm. Avantageusement, un diamètre de 1200 µm permet une insertion correcte dans un tube guide existant.

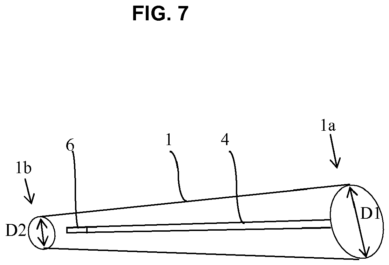

De préférence, la section transversale de la tige 1 décroit entre son extrémité proximale et son extrémité distale. Par exemple, comme illustré sur la figure 7, lorsque la tige présente une section circulaire, le diamètre D2 de l'extrémité distale est inférieur au diamètre D1 de l'extrémité proximale. De préférence, le diamètre (ou la diagonale lorsque la section n'est pas circulaire) de l'extrémité distale est trois fois inférieur au diamètre (ou la diagonale) de l'extrémité proximale. Cela permet une meilleure prise en main de la tige par un opérateur, au niveau de l'extrémité proximale.

La tige 1 porte une surface de capture 2 qui est destinée à être appliquée contre un tissu biologique au niveau duquel on souhaite effectuer un prélèvement.

La surface de capture 2 est généralement une surface plane et de contour rectangulaire, bien qu'elle ne soit pas limitée à cette seule forme. La longueur de la surface de capture est de préférence agencée parallèlement à l'axe longitudinal.

La tige 1 peut présenter un méplat agencé à la périphérie de la tige et sur lequel on solidarise la surface de capture, par exemple par collage ou par tout autre moyen. La liaison de la surface de capture vis-à-vis de la tige peut être amovible ou non. Le méplat est avantageusement conçu pour que lorsque la surface de capture 2 est assemblée sur la tige 1, elle soit inscrite dans la section circulaire de la tige et ne présente pas de protubérance par rapport à la surface périphérique de la tige.

Tout en étant plane à l'échelle macroscopique, la surface de capture peut être micro-structurée de sorte à présenter des protubérances permettant de prélever un tissu biologique par micro-abrasion. Par exemple, lesdites protubérances peuvent consister en des piliers hexagonaux de 50 µm de hauteur et 80 µm de diamètre.

De manière alternative, la surface de capture peut être la surface d'un matériau nanoporeux.

Le silicium est fréquemment employé comme matériau de surface de capture, quel que soit le mode de réalisation considéré.

Par ailleurs, la surface de capture 2 peut être revêtue d'une couche de fonctionnalisation favorisant le greffage d'analytes d'intérêt. Dans le présent texte, le terme « analyte » désigne une espèce chimique ou biologique, en particulier une protéine ou une cellule. La couche de fonctionnalisation peut notamment être anionique.

De préférence, la surface de capture 2 est située dans une région distale de la tige 1.

Par ailleurs, la tige 1 comprend une fenêtre d'observation 3 et un logement 4 s'étendant dans la tige depuis l'extrémité proximale 1a jusqu'à ladite fenêtre d'observation. Comme on le verra en détail plus bas, ledit logement 4 est destiné à recevoir un faisceau de fibres optiques.

Par « fenêtre » on entend dans le présent texte une surface adaptée pour transmettre la lumière visible entre l'environnement extérieur et le logement 4. Ladite fenêtre est donc obtenue en pratiquant une ouverture entre la surface externe de la tige et le logement 4. La fenêtre peut éventuellement être matérialisée par un matériau transparent à la lumière visible, ce matériau pouvant être organique (tel que du plexiglas) ou inorganique (tel que du verre). Cependant, la présence d'un tel matériau n'est pas indispensable et la fenêtre peut simplement consister en un espace libre entre la surface externe de la tige 1 et le logement 4.

Selon un mode de réalisation, le logement 4 peut consister en un perçage pratiqué dans l'épaisseur de la tige.

De manière alternative, le logement 4 peut consister en une rainure pratiquée dans la surface externe de la tige 1, qui est généralement plus facile à réaliser par usinage. Ladite rainure peut présenter par exemple une section carrée ou rectangulaire.

Les dimensions de ladite rainure sont adaptées à celles du faisceau de fibres optiques, de sorte à permettre l'insertion dudit faisceau dans ladite rainure. De préférence, le faisceau de fibres optiques est agencé de manière amovible dans le logement ; il est par exemple retenu par serrage dans le logement 4. Le faisceau peut être inséré partiellement dans la rainure, c'est-à-dire qu'une partie du faisceau s'étend au-delà des parois de la rainure ; de manière alternative, le faisceau est entièrement logé dans la rainure, sa surface externe ne dépassant pas de l'enveloppe définie par les parois de la rainure.

Lorsque le faisceau de fibres optiques est inséré en totalité dans la rainure, l'espace laissé libre dans la rainure autour du faisceau peut être comblé au moyen d'un polymère biocompatible appliqué à l'état liquide puis durci de manière à constituer un confinement du faisceau de fibres. De préférence, ledit polymère assure en outre une continuité de la surface externe de la tige pour conserver sa section circulaire. Ledit polymère est de préférence un alginate, qui est biocompatible et permet un comblement réversible de la rainure. En effet, cet alginate peut ensuite être dissous dans un bain d'un solvant approprié, ce qui permet de retirer le faisceau de fibres optiques de la rainure.

Le faisceau de fibres optiques 5 comprend avantageusement de l'ordre de 3000 fibres optiques agencées en un faisceau de section circulaire de 0,3 mm de diamètre. Un tel faisceau est celui équipant l'endoscope distribué par la société Mauna Kea Technologies sous la référence Cellvizio™.

Les fibres optiques sont choisies de sorte à guider toute longueur d'onde permettant la caractérisation spectrale des tissus biologiques. Typiquement, les fibres optiques doivent guider une plage de longueur d'onde comprise entre l'ultraviolet et l'infrarouge, ce dernier présentant une bien meilleure pénétrance dans les tissus.

Le faisceau 5 présente une extrémité proximale qui est reliée à un système d'excitation et de détection, et une extrémité distale qui, lorsque le faisceau est dans sa position d'utilisation dans la tige 1, est agencée au voisinage de la fenêtre d'observation 3.

Le dispositif 100 comprend par ailleurs un système optique de renvoi 6 adapté pour réaliser un couplage optique entre l'extrémité distale du faisceau et la fenêtre d'observation. A cet effet, ledit système 6 est agencé au voisinage de la fenêtre 3. Le système de couplage optique peut être solidaire de la tige (il peut par exemple être formé d'un prisme collé à l'extrémité distale de la rainure formant le logement 4, en vis-à-vis de la fenêtre d'observation 3) ou bien du faisceau de fibres, auquel cas il est fixé à l'extrémité distale de ce faisceau.

Selon un mode de réalisation, le système de couplage optique est constitué par l'extrémité du faisceau qui est taillée en biseau, l'angle du biseau étant de l'ordre de 40 à 50°, de préférence 45°. Selon ce mode de réalisation, l'extrémité du faisceau forme un plan incliné ; ainsi, chaque fibre du faisceau est apte à collecter un signal lumineux dont l'angle d'incidence est incliné par rapport à l'axe longitudinal du faisceau.

Selon un autre mode de réalisation, le système de renvoi optique est une lame réfléchissante, orientée d'environ 40 à 50°, de préférence 45°, par rapport à l'axe longitudinal X.

Ladite lame réfléchissante peut appartenir à un prisme, ledit prisme étant solidaire de l'extrémité distale du faisceau de fibres optiques, par exemple par sertissage, par collage ou par tout autre moyen. Le prisme présente typiquement une section carrée dont la longueur est égale au diamètre du faisceau de fibres optiques.

De préférence, le système de renvoi optique est configuré de façon à dévier la lumière émise par le faisceau de fibres selon une direction sensiblement orthogonale à l'axe longitudinal X. Dans ce cas, le faisceau est apte à collecter, par son extrémité distale, une lumière dont l'angle d'incidence est orthogonal à l'axe longitudinal X.

Le dispositif 100 comprend avantageusement un tube guide 7 à l'intérieur duquel la tige 1 peut coulisser. Le tube guide comprend une ouverture latérale 70 de telle sorte que lorsque la tige est insérée dans le tube, l'ouverture 70 est en vis-à-vis de la portion de la tige qui porte la surface de capture et dans laquelle est ménagée la fenêtre d'observation 3. La tige 1 est alors libre en translation et en rotation dans le tube guide 7. Cela permet de placer, alternativement, la fenêtre 3 ou la surface de capture 2 face à l'ouverture 70, comme détaillé dans l'exemple décrit ci-après. Le tube guide 7 a pour fonction de protéger la tige et la surface de capture, qui peuvent coulisser dans le tube sans être en contact avec les tissus. Le tube guide est en un matériau biocompatible tel que de l'inox. Le diamètre intérieur du tube guide est typiquement compris entre 1000 et 3000 µm, par exemple 1200 µm.

Lorsque la tige présente une section décroissante de son extrémité proximale vers son extrémité distale (mode de réalisation illustré sur la figure 7), la forme du tube guide peut être adaptée en conséquence. Ainsi, l'ouverture du tube guide, au niveau de son extrémité proximale, est plus large qu'au niveau de son extrémité distale. La géométrie externe du tube guide peut également être discontinue, le diamètre (ou la diagonale dans le cas d'une section non circulaire) externe de l'extrémité proximale étant supérieur au diamètre (ou la diagonale) au niveau de l'extrémité distale.

La figure 2 est une vue de face de l'extrémité proximale de la tige 1.

Le logement 4 est dans ce cas une rainure de section rectangulaire dans laquelle le faisceau de fibres optiques 5 est entièrement contenu. Un prisme 6 de base carrée solidaire du faisceau est agencé à l'extrémité distale de la rainure, en vis-à-vis de la fenêtre d'observation (non représentée ici). Diamétralement opposé à la rainure 4 est agencé un méplat portant la surface de capture 2, qui est ici micro-structurée au moyen de plots saillants.

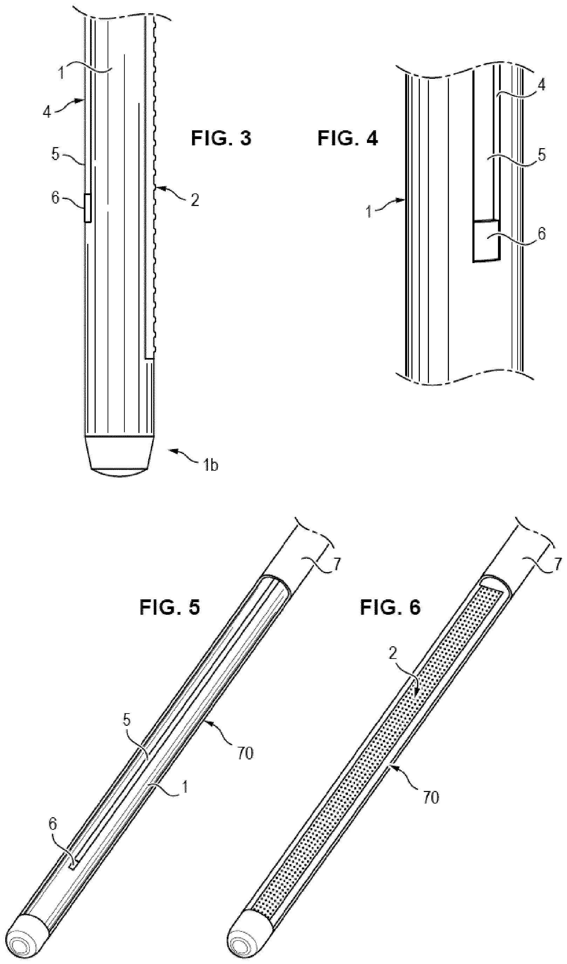

La figure 3 est une vue de côté de la tige, le logement 4 étant une rainure diamétralement opposée à la surface de capture 2.

La figure 4 est une vue de l'extrémité distale de la rainure, qui contient le faisceau 5 et un système optique de renvoi 6 agencée à l'extrémité du faisceau.

Revenant maintenant à la figure 1, le système d'excitation et de détection comprend typiquement une source de lumière L (par exemple un laser) et un photodétecteur D (de préférence un photodétecteur matriciel). La source de lumière émet une lumière à une longueur d'onde d'excitation. Le photodétecteur est agencé pour détecter une lumière à une longueur d'onde d'émission, cette dernière étant notamment produite par un tissu biologique en réponse à la lumière d'excitation. L'extrémité proximale du faisceau, la source de lumière et le photodétecteur sont avantageusement couplés par un système optique de couplage C, par exemple un miroir dichroïque.

Le photodétecteur est de préférence couplé à un filtre, par exemple un filtre interférentiel, dont la bande passante est centrée sur la longueur d'onde de fluorescence (longueur d'onde d'émission). Ce filtre peut être disposé sur le miroir dichroïque, ou entre ce dernier et le photodétecteur.

Pour la mise en oeuvre d'une opération de prélèvement, on agence la tige 1 dans le tube guide 7 de sorte que la fenêtre d'observation soit en vis-à-vis de l'ouverture 70 (cf. figure 5).

Le faisceau étant en position dans son logement de la tige, les fibres optiques collectent le signal lumineux d'excitation émis par la source lumineuse L qui est couplée à l'extrémité proximale du faisceau jusqu'à l'extrémité distale du faisceau. Le signal est dévié par le système de renvoi optique 6 et transmis vers l'environnement extérieur du tube à travers la fenêtre d'observation 3. Ainsi, le signal vient exciter des marqueurs fluorescents qui ont préalablement été injectés au patient qui doit subir le prélèvement, ou des marqueurs fluorescents endogènes, naturellement présents dans les tissus biologiques.

Lesdits marqueurs émettent alors un signal de fluorescence, qui est transmis au travers de la fenêtre d'observation 3, dévié par le système de renvoi optique 6 et conduit par les fibres optiques jusqu'au photodétecteur D au travers du système optique de couplage C, selon le trajet inverse de celui du signal d'excitation.

Le faisceau de fibres optiques assure ainsi deux fonctions :
- d'une part, l'illumination des tissus éclairés au travers de la fenêtre d'observation 3 ;
- d'autre part, la collection d'un signal de fluorescence émis par les tissus éclairés.

Le photodétecteur est adapté pour détecter un signal de fluorescence émis par les tissus et par conséquent indicateur de la présence ou non des tissus d'intérêt, ces derniers générant un signal de fluorescence détectable par le photodétecteur D.

Si l'analyse effectuée par le photodétecteur montre que le dispositif est situé dans une région propice, le praticien amène la surface de capture 2 en vis-à-vis de l'ouverture 70 pour la mettre au contact des tissus (cf. figure 6).

De préférence, la fenêtre d'observation 3 est située à la même position longitudinale de la tige que la surface de capture mais décalée angulairement. Ce décalage angulaire est par exemple de 180° (la surface de capture et la fenêtre d'observation étant diamétralement opposées) mais tout autre angle peut être choisi. De manière avantageuse, le dispositif comprend un système d'indexation angulaire qui lui permet de positionner aisément soit la fenêtre d'observation 3 soit la surface de capture 2 en vis-à-vis de l'ouverture 70 du tube guide 7. Ainsi, une fois que le dispositif est en place dans une région propice au prélèvement, le praticien immobilise le tube guide 7 et se contente de faire pivoter la tige 1 pour amener la surface de capture 2. Il est ainsi certain d'effectuer le prélèvement dans la même région que celle qu'il a observée et choisie.

Le cas échéant, plusieurs prélèvements peuvent être effectués dans la même région : il suffit à cet effet de retirer du tube guide la tige portant la surface de capture sur laquelle un échantillon a été prélevé et d'insérer une nouvelle tige portant une surface de capture vierge.

Par ailleurs, une fois que le tube guide a été positionnée dans la région souhaitée, il est possible de retirer le faisceau de fibres optiques de la tige et d'insérer dans le tube guide la tige pourvue uniquement de la surface de capture.

Enfin, on a mentionné ci-dessus la manipulation du dispositif de prélèvement par un chirurgien mais il est également possible que le dispositif soit manipulé par un robot.

## Revendications

1. Dispositif (100) de prélèvement d'un tissu biologique comprenant :
- une tige (1) s'étendant selon un axe longitudinal (X) entre une extrémité proximale (1a) et une extrémité distale (1b),
- une surface de capture (2) portée par la tige (1), destinée à être appliquée contre un tissu biologique,
- une fenêtre d'observation (3) transparente à la lumière visible, agencée dans la surface externe de la tige (1),
- un logement (4) s'étendant dans la tige, parallèlement à l'axe longitudinal, de l'extrémité proximale (1a) jusqu'à la fenêtre d'observation (3), ledit logement étant apte à recevoir un faisceau de fibres optiques,
ledit dispositif (100) étant **caractérisé en ce que** la surface de capture est nanoporeuse ou présente des protubérances adaptées pour prélever un tissu biologique par microabrasion et **en ce que** le logement (4) pour le faisceau de fibres optiques comprend une rainure agencée dans la surface externe de la tige.

2. Dispositif selon la revendication 1, dans lequel la fenêtre d'observation (3) et la surface de capture (2) sont agencées à une même distance de l'extrémité distale de la tige et angulairement espacées.

3. Dispositif selon l'une des revendications 1 à 2, comprenant :
- un faisceau (5) de fibres optiques disposé dans le logement (4) de la tige de telle sorte qu'une extrémité distale dudit faisceau soit agencée au voisinage de la fenêtre d'observation (3) et
- un système optique de renvoi (6) adapté pour réaliser un couplage optique entre l'extrémité distale du faisceau et la fenêtre d'observation.

4. Dispositif selon la revendication 3, dans lequel le système optique de renvoi comprend une lame réfléchissante orientée d'un angle compris entre 40 et 50° par rapport à l'axe longitudinal (X) de la tige.

5. Dispositif selon la revendication 4, dans lequel la lame réfléchissante fait partie d'un prisme.

6. Dispositif selon la revendication 5, dans lequel le prisme comprenant la lame réfléchissante est solidaire de l'extrémité distale du faisceau de fibres optiques.

7. Dispositif selon la revendication 3, dans lequel le système optique de renvoi consiste en une extrémité distale biseautée des fibres optiques formant le faisceau.

8. Dispositif selon l'une des revendications 1 à 7, comprenant en outre un tube guide (7) dans lequel la tige (1) est apte à coulisser, ledit tube guide comprenant une ouverture latérale (70) agencée de telle sorte que lorsque la tige est introduite dans le tube guide la surface de capture et la fenêtre d'observation viennent en vis-à-vis de ladite ouverture latérale (70) selon l'orientation angulaire respective du tube guide et de la tige.

9. Dispositif selon la revendication précédente, comprenant en outre un système d'indexation angulaire permettant de positionner soit la surface de capture (2) soit la lumière d'observation (3) en vis-à-vis de l'ouverture latérale (70) du tube guide (7).

10. Dispositif selon l'une des revendications 1 à 9, dans lequel la surface de capture est située dans une région distale de la tige (1).

11. Dispositif selon l'une des revendications 1 à 10, dans lequel la surface de capture est revêtue d'une couche de fonctionnalisation.

12. Dispositif selon l'une des revendications 1 à 11, dans lequel la tige (1) est en inox ou en PEEK.

13. Dispositif selon l'une des revendications 1 à 12, dans lequel la tige (1) présente une section décroissante de son extrémité proximale (1a) vers son extrémité distale (1b).

## Patentansprüche

1. Vorrichtung (100) zur Probenahme von biologischem Gewebe, umfassend:
- einen Stab (1), der sich entlang einer Längsachse (X) zwischen einem proximalen Ende (1a) und einem distalen Ende (1b) erstreckt,
- eine vom Stab (1) getragene Auffangfläche (2), die dazu bestimmt ist, gegen ein biologisches Gewebe aufgebracht zu werden,
- ein für sichtbares Licht transparentes Beobachtungsfenster (3), das in der Außenfläche des Stabes (1) gestaltet ist,
- ein Gehäuse (4), das sich im Stab parallel zur Längsachse vom proximalen Ende (1a) bis zum Beobachtungsfenster (3) erstreckt, wobei das Gehäuse geeignet ist, ein optisches Faserbündel aufzunehmen,
wobei die Vorrichtung (100) **dadurch gekennzeichnet ist, dass** die Auffangfläche nanoporös ist oder Vorsprünge aufweist, die geeignet sind, ein biologisches Gewebe durch Mikroabrieb zu entnehmen, und dass das Gehäuse (4) für das optische Faserbündel eine Nut aufweist, die in der Außenfläche des Stabes gestaltet ist.

2. Vorrichtung nach Anspruch 1, wobei das Beobachtungsfenster (3) und die Auffangfläche (2) im gleichen Abstand vom distalen Ende des Stabes und im Winkelabstand angeordnet sind.

3. Vorrichtung nach einem der Ansprüche 1 bis 2, umfassend:
- ein optisches Faserbündel (5), das im Gehäuse (4) des Stabes so angeordnet ist, dass ein distales Ende des Bündels in der Nähe des Beobachtungsfensters (3) gestaltet ist, und
- ein optisches Umlenksystem (6), das geeignet ist, eine optische Kopplung zwischen dem distalen Ende des Bündels und dem Beobachtungsfenster durchzuführen.

4. Vorrichtung nach Anspruch 3, wobei das optische Umlenksystem eine reflektierende Lamelle umfasst, die in einem Winkel zwischen 40 ° und 50 ° zur Längsachse (X) des Stabes ausgerichtet ist.

5. Vorrichtung nach Anspruch 4, wobei die reflektierende Lamelle Teil eines Prismas ist.

6. Vorrichtung nach Anspruch 5, wobei das Prisma, das die reflektierende Lamelle umfasst, fest mit dem distalen Ende des optischen Faserbündels verbunden ist.

7. Vorrichtung nach Anspruch 3, wobei das optische Umlenksystem aus einem abgeschrägten distalen Ende der das Bündel bildenden optischen Fasern besteht.

8. Vorrichtung nach einem der Ansprüche 1 bis 7, ferner umfassend ein Führungsrohr (7), in dem der Stab (1) verschiebbar ist, wobei das Führungsrohr eine seitliche Öffnung (70) umfasst, die so gestaltet ist, dass, wenn der Stab in das Führungsrohr eingeführt wird, die Auffangfläche und das Beobachtungsfenster der seitlichen Öffnung (70) entsprechend der jeweiligen Winkelausrichtung des Führungsrohrs und des Stabes zugewandt sind.

9. Vorrichtung nach dem vorstehenden Anspruch, ferner umfassend ein Winkelindexsystem zum Positionieren entweder der Auffangfläche (2) oder des Beobachtungslichts (3) gegenüber der seitlichen Öffnung (70) des Führungsrohrs (7).

10. Vorrichtung nach einem der Ansprüche 1 bis 9, wobei sich die Auffangfläche in einem distalen Bereich des Stabes (1) befindet.

11. Vorrichtung nach einem der Ansprüche 1 bis 10, wobei die Auffangfläche mit einer Funktionalisierungsschicht versehen ist.

12. Vorrichtung nach einem der Ansprüche 1 bis 11, wobei der Stab (1) aus Edelstahl oder aus PEEK besteht.

13. Vorrichtung nach einem der Ansprüche 1 bis 12, wobei der Stab (1) einen sich verjüngenden Abschnitt von seinem proximalen Ende (1a) zu seinem distalen Ende (1b) aufweist.

## Claims

1. A device (100) for sampling a biological tissue comprising:
- a rod (1) extending along a longitudinal axis (X) between a proximal end (1a) and a distal end (1b),
- a capture surface (2) borne by the rod (1), intended to be applied against a biological tissue,
- an observation window (3) transparent to visible light, arranged in the external surface of the rod (1),
- a housing (4) extending in the rod, parallel to the longitudinal axis, from the proximal end (1a) as far as the observation window (3), said housing being able to receive a bundle of optical fibres,
said device (100) being **characterized in that** the capture surface is nanoporous or has protrusions adapted for sampling a biological tissue by micro abrasion and **in that** the housing (4) for the bundle of optical fibres comprises a groove arranged in the external surface of the rod.

2. The device according to claim 1, wherein the observation window (3) and the capture surface (2) are arranged at a same distance from the distal end of the rod and spaced out angularly.

3. The device according to one of claims 1 to 2, comprising:
- a bundle (5) of optical fibres positioned in the housing (4) of the rod so that a distal end of said bundle is arranged in the vicinity of the observation window (3) and
- an optical return system (6) adapted for carrying out optical coupling between the distal end of the bundle and the observation window.

4. The device according to claim 3, wherein the optical return system comprises a reflective plate orientated at an angle comprised between 40 and 50° relative to the longitudinal axis (X) of the rod.

5. The device according to claim 4, wherein the reflective plate is part of a prism.

6. The device according to claim 5, wherein the prism comprising the reflective plate is secured to the distal end of the bundle of optical fibres.

7. The device according to claim 3, wherein the optical return system consists in a bevelled distal end of the optical fibres forming the bundle.

8. The device according to one of claims 1 to 7, further comprising a guide tube (7) in which the rod (1) is able to slide, said guide tube comprising a lateral opening (70) arranged so that, when the rod is introduced into the guide tube, the capture surface and the observation window are facing said lateral opening (70) depending on the respective angular orientation of the guide tube and of the rod.

9. The device according to the previous claim, further comprising an angular indexation system which allows positioning either the capture surface (2) or the observation window (3) facing the lateral opening (70) of the guide tube (7).

10. The device according to one of claims 1 to 9, wherein the capture surface is located in a distal region of the rod (1).

11. The device according to one of claims 1 to 10, wherein the capture surface is coated with a functionalization layer.

12. The device according to one of claims 1 to 11, wherein the rod (1) is in stainless steel or in PEEK.

13. The device according to one of claims 1 to 12, wherein the rod (1) has a decreasing section from its proximal end (1a) to its distal end (1b).
